# EUROPEAN PATENT APPLICATION

(11) **EP 2 202 234 A1**
(43) Date of publication of application: **30.06.2010**
(21) Application number: 08172904.8
(22) Date of filing: 24.12.2008
(51) Int. Cl.: C07D 471/04

(54) **Purification of paliperidone**

(71) Applicant: Laboratorios Lesvi, S.L., 08970 Sant Joan Despí - Barcelona (ES)
(72) Inventor: Huguet Clotet, Juan, 08970 Sant Joan Despí (ES); Calcerrada Muñoz, Noelia, 08970 Sant Joan Despí (ES)

(57) **Abstract**

The present invention relates to a process for the purification of Paliperidone from its impurities by a simple process that can be used at industrial scale, with overall good yields, which reduces the number of crystallisation steps and removes all impurities, yielding Paliperidone of very high purity. Also, the present invention relates to the preparation of pure Paliperidone *via* Paliperidone salts.

## Description

### Field of invention

The present invention relates to a process for the purification of Paliperidone from its impurities. Also, the present invention relates to the preparation of pure Paliperidone *via* Paliperidone salts.

### Background of the invention

Paliperidone is a metabolite of Risperidone, marketed under the name of Invega®. Paliperidone is a psychotropic agent, approved for the treatment of schizophrenia. The chemical name for Paliperidone is (±)-3-[2-[4-(6-fluoro-1,2-benzisoxazol-3-yl)-1-piperidinyl]ethyl]-6,7,8,9-tetrahydro-9-hydroxy-2-methyl-4*H-*pyrido[1,2-a]pyrimidin-4-one, and its chemical structure is shown below:

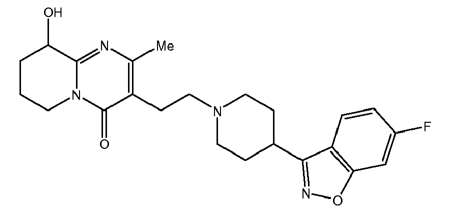

Paliperidone was first disclosed in European patent EP 368.388 B1 in said application Paliperidone is prepared as depicted in scheme 1.

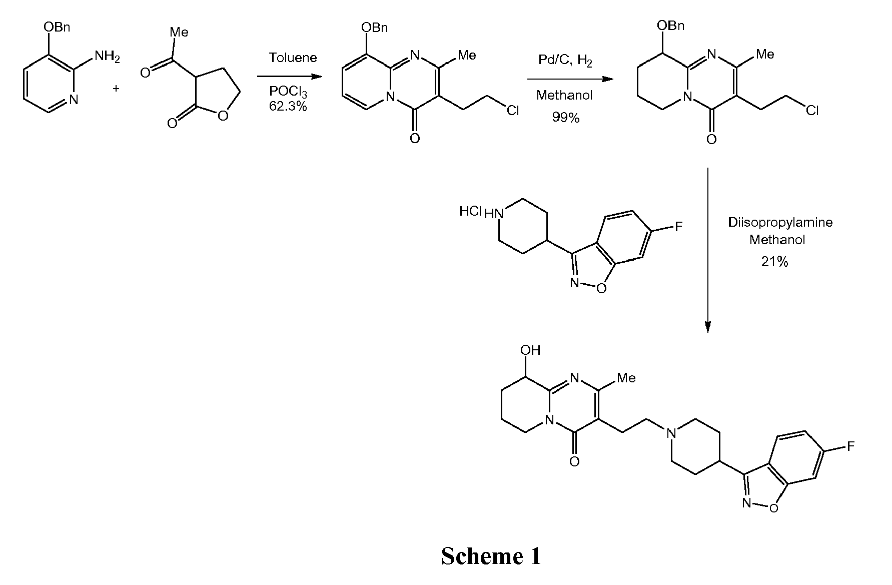

Another process for the synthesis of a precursor of Paliperidone, (3-(2-chloroethyl)-2-methyl-9-benzyloxy-4H-pyrido[1,2-a]-pyrimidine-4-one), is described in European patent EP 1.945.640 A . Paliperidone is obtained *via* the intermediate 3-(2-chloroethyl)- 6,7,8,9-tetrahydro-9-hydroxy-2-methyl-4H-pyrrido[1,2-a]-pyrimidin-4-one (CMHTP) in the presence of an organic base.

Alternatively Paliperidone can be prepared following the process described in unpublished application EP08157224.0 whereby Paliperidone is synthesised via intermediates, which do not bear protecting groups, achieving higher yields, an increase in the atomic economy and less environmental drawbacks.

PCT No W02008/087557 describes an improved process for preparation of 9-hydroxy-3-2-chloroethyl)-2-methyl-4h-pyrido[1,2-a]pyrimidin-4-one, an important intermediate for the preparation of Paliperidone.

US Patent Nos. 5,254,556 and 5,688,799 also describe the preparation of Paliperidone and Risperidone related compounds. Paliperidone obtained by the process described in the '556 and the '799 patents does not yield satisfactory purity. Significant amounts of impurities are generally formed during the reaction between the 2-aminopyridine compound and the sigma-acyl lactone compound when the reaction is carried out in the presence of solvents like toluene, thus resulting in a poor product yield. Similarly to patents '556 and the '799, the European Patent EP 368.388 B1 also involves the purification of intermediary compounds by means of column chromatography acquiring low yields. Methods involving column chromatographic purifications are generally undesirable for large-scale operations, thereby making the process commercially unfeasible.

Impurities present in Paliperidone may be un-reacted starting materials, byproducts of the reaction, products of side reactions, or degradation products. At certain stages during processing of the active ingredient, Paliperidone must be analyzed for purity, typically by standard characterisation techniques such as, HPLC, TLC or GC, in order to determine if it is suitable for continued processing and, ultimately, for use in a pharmaceutical product. In fact the United States Food and Drug Administration guidelines recommend that the amounts of some impurities be limited to less than 0.1 percent.

US Patent application No 2008171876, describes a method for the purification of Paliperidone, which was previously obtained *via* intermediate 3-(2-chloroethyl)-6,7,8,9-tetrahydro-9-hydroxy-2-methyl-4H-pyrrido[1,2-a]-pyrimidin-4-one. Purification of Paliperidone is carried out by means of re-crystallization using organic solvents or a mixture of organic and inorganic solvents with the purpose of removing two potential known impurities, either PLP-NO or PLP-Car. However, the process described therein, does not eliminate both impurities at the same time, and therefore, at least two re-crystallisations will be needed.

Thus, it is still required an efficient method for the purification of Paliperidone that eliminates impurities independent from its origin, with good yields, that can be used at industrial scale.

### Summary of the invention

The inventors have found a purification process of crude Paliperidone by means of obtaining a Paliperidone salt that further provides pure Paliperidone and removes known and unknown impurities, which may be present in the crude Paliperidone.

The first embodiment of the present invention provides a process for the purification of Paliperidone, yielding pure Paliperidone with a total purity of at least about 99.2%, preferably 99,5%, most preferably 99.9%.

In a second embodiment, the present invention relates to Paliperidone salts and to a process for the preparation of Paliperidone salts.

### Detailed description of the invention

As used herein, "pure Paliperidone" refers to Paliperidone containing less than about 1% total impurity content.

The term "reduced pressure" refers to a pressure less than 100 mm Hg

Paliperidone salt is also referred as to compound of formula **II** and is characterised by the structure below:

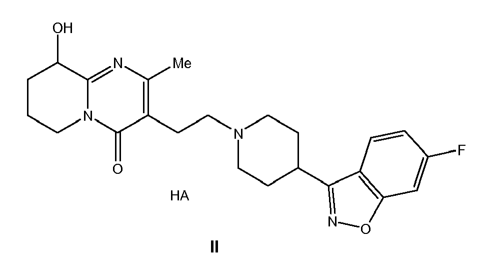

Whereby HA is an organic or an inorganic acid selected from the group consisting of: hydrochloric acid, hydrobromic acid, sulfuric acid, nitric acid, phosphoric acid, acetic acid, citric acid, formic acid, gluconic acid, lactic acid, oxalic acid, tartaric acid.

The inventors have observed that the methods known in the art for the synthesis of Paliperidone leads to the formation of crude Paliperidone, with purity levels of about 95 %, that has to be purified in order to be used in pharmaceutical compositions. One of the purification methods described in patent EP 368.388 B1 involves purifying crude Paliperidone by column chromatography. Further to the column chromatography treatment Paliperidone is also re-crystallised in isopropyl alcohol (IPA). It has been discovered by the inventors that re-crystallisation of Paliperidone in IPA promotes the growth of an impurity which has been
characterised as 3-{2-[4-(6-Fluoro-benzo[d]isoxazol-3-yl)-piperidin-1-yl]-ethyl}-2-methyl-7,8-dihydro-6H-pyrido[1,2-a]pyrimidine-4,9-dione (also referred as to PLP-ceto). Other name for the PLP-ceto impurity is 9-oxo-Risperidone. The growth of PLP-ceto is caused by the high temperature (80-85 °C) needed to dissolve Paliperidone in isopropyl alcohol. As a result of that, multiple re-crystallisations as well as high quantities of IPA are needed in order to minimise and eliminate the impurities present in crude Paliperidone and to minimise, however not completely remove PLP-Ceto.

PLP-Ceto is has been characterised by means of conventional characterisation methods such as ¹H NMR, FTIR, Mass spectrometry and HPLC. Chemical structure of PLP-Ceto is shown below

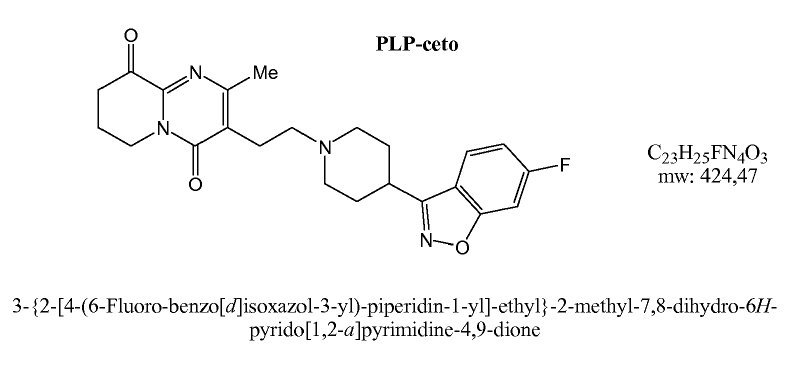

Thus, crystallisation methods are not efficient enough to purify Paliperidone at an industrial scale since several crystallisation steps as well as the use of high quantities of different solvents are required in order to eliminate all the impurities.

The present inventors have surprisingly found a simple process for the purification of Paliperidone that can be used at industrial scale, with overall good yields, which reduces the number of crystallisation steps and removes all impurities, yielding Paliperidone of very high purity, at least about 99.2%, preferably 99.5%, more preferably 99.9 %. The percentage of each impurity does not exceed 0.05%, a very satisfactory figure compared to that recommended by FDA guidelines of 0.1%. The obtained Paliperidone has a very appropriate purity for pharmaceutical purposes.

The first embodiment of the present invention provides a process for the purification of Paliperidone. This process comprises the steps of:
A- Forming a Paliperidone salt from Paliperidone base by addition of an acid to crude Paliperidone base.
B- Optionally, recovering Paliperidone salt of formula **II**
C- Neutralising Paliperidone salt by treating with a base until a pH between about 9-10 is reached,
D- Extracting Paliperidone base with organic solvent,
E- Collecting Paliperidone,
F- Optionally washing, re-precipitating or re-crystallising the purified Paliperidone

The first step (step A) comprises the formation of a Paliperidone salt from Paliperidone base by reacting crude Paliperidone base with an acid. Crude Paliperidone can be prepared following the process described in unpublished application EP08157224.0 having a purity of between 97-99%. Crude Paliperidone can be used directly as obtained from previous synthetic steps (as an oil, solid, paste, dispersion...) or may be dissolved or suspended in a polar or an apolar organic solvent, an aqueous solution or a mixture thereof. The organic solvent is selected from aromatic organic solvents, chloro-compounds, ketones, esters, ethers, and mixtures thereof. More preferably the solvent is water.

The acid may be an inorganic acid or an organic acid, either concentrated or diluted in an organic solvent, an aqueous solution or mixtures thereof. Preferably, the inorganic acid is selected from hydrochloric acid, hydrobromic acid, sulfuric acid, nitric acid, phosphoric acid and chromic acid, preferably hydrochloric acid. In a preferred embodiment the inorganic acid is hydrochloric acid. Hydrochloric acid can be used concentrated or diluted as aqueous solution or methanolic solution having a concentration from 0.1 Molar to 12 M. Also preferably, the organic acid is selected from acetic acid, citric acid, formic acid, gluconic acid, lactic acid, oxalic acid, tartaric acid and mixtures thereof. Preferably the organic acids are acetic acid and formic acid.

The formation of the Paliperidone salt (step A) is carried out at temperatures from -10°C to 60°C, preferably at a temperature of about 0 °C to about 20°C. More preferably, at a temperature of about 0 °C to about 10°C. The obtained salt of Paliperidone can be kept in solution or in suspension, and further processed according to step C of the present invention. Optionally, the Paliperidone salt can be collected and isolated (step B) by conventional methods, such as, precipitation, extraction, filtration, centrifugation or by decanting. The obtained compound of formula **II** is then optionally subjected to drying, using conventional drying techniques, like vacuum oven drying.

The mass of step A, which contains Paliperidone salt, is neutralised (step C) with an organic or an inorganic base until a pH about 9-10 is reached. Alternatively, the isolated (step B) solid Paliperidone salt is dissolved or suspended in an organic or an aqueous solvent, or mixtures thereof, and neutralised with an organic or an inorganic base until a pH about 9-10 is reached. Preferably, the inorganic base is selected from alkoxides or carbonates of alkaline metals or mixtures thereof. In a preferred embodiment, the base is aqueous sodium hydroxide. Also preferably, the organic base is selected from ammonia, n-butyl amine, pyrrolidine, diethyl amine, morpholine, piperazine, di-isopropyl amine, triethyl amine, diisopropylethyl amine (DIPEA) and mixtures thereof. Also, the base can be a mixture of any of the inorganic and organic aforementioned bases.

Step C is **characterised in that** the reaction is carried out in a solvent system. The solvent system comprises a first solvent and a second solvent. The first solvent being an organic solvent and the second solvent a non-miscible or partially miscible solvent, which can be an organic solvent, an aqueous solution or mixtures thereof. The preferred first organic solvent is selected from aromatic organic solvents, chloro-compounds, ketones, esters, ethers or mixtures thereof. The preferred second solvent is water.

Following the neutralisation step, the obtained product, Paliperidone base, is preferably recovered by extraction with an organic solvent (Step D). The preferred organic solvent is any of those described in step C.

Optionally, the organic phase is separated and treated with activated carbon for a period between 15 min and 2 hours, at temperature range of about 20° up to 80 °C. Afterwards the organic phase is filtered.

The pure Paliperidone obtained in step D (with a purity ranging from 99.1-99.8 %) is collected by filtration or centrifugation. Said pure compound is then optionally subjected to washing, re-crystallising and drying using conventional drying techniques like vacuum oven drying.

The purified Paliperidone is optionally subjected to re-crystallisation by means of isopropyl alcohol, ethanol, methanol, water, ketones, THF or mixture thereof. The solid obtained has a purity of about 99.9%.

The second embodiment of the present invention provides the preparation of a Paliperidone salt. Said Paliperidone salt can be isolated, purified, characterised and stored. Paliperidone salt is also referred as to compound of formula **II** and is characterised by the structure below:

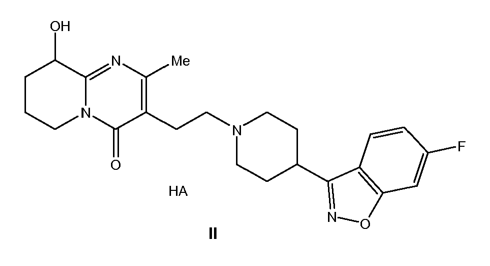

Whereby HA is an organic or inorganic acid, preferably the acid is selected from the group consisting of: hydrochloric acid, hydrobromic acid, sulfuric acid, nitric acid, phosphoric acid, acetic acid, citric acid, formic acid, gluconic acid, lactic acid, oxalic acid, tartaric acid.

Thus, Paliperidone salt can be prepared according to steps A and B of the process which comprises the following steps:
A.- Crude Paliperidone is dissolved or suspended in a solvent system
B.- An acid is added to the solution of step A
C.- The solid formed is separated

Crude Paliperidone can be used directly as obtained from previous synthetic steps (as an oil, solid, paste, dispersion, etc.) or may be suspended or dissolved in a polar or in a non-polar organic solvent, an aqueous solution or a mixture thereof. The organic solvent is selected from, chloro-compounds, aromatic organic solvents, ketones, esters, ethers, and mixtures thereof. More preferably the solvent is water.

The acid may be an inorganic acid or an organic acid, either concentrated or diluted in an organic solvent, an aqueous solution or mixtures thereof. Preferably, the inorganic acid is selected from hydrochloric acid, hydrobromic acid, sulfuric acid, nitric acid, phosphoric acid and chromic acid. In a preferred embodiment the inorganic acid is hydrochloric acid. Hydrochloric acid can be used concentrated or diluted as aqueous solution or alcoholic solution having a concentration from 0.1 Molar to 12 M. Also preferably, the organic acid is selected from acetic acid, citric acid, formic acid, fumaric acid, gluconic acid, lactic acid, oxalic acid, tartaric acid and mixtures thereof. Preferably the organic acids are acetic acid and formic acid.

The formation of the Paliperidone salt (step A) is carried out at temperatures from -10°C to 60°C, preferably at a temperature of about 0 °C to about 20°C

Optionally, the Paliperidone salt can be separated and isolated by conventional methods, such as precipitation, extraction, filtration, centrifugation or by decanting. The obtained compound of formula **II** is then optionally subjected to drying, using conventional drying techniques, like vacuum oven drying.

In a preferred embodiment, the salt obtained is Paliperidone hydrochloride. Crude Paliperidone is treated with hydrochloric acid. Preferably, concentrated Hydrochloric acid (37%) is used. The obtained salt of Paliperidone can be transformed into pure Paliperidone base following the process described above.

The Paliperidone salt can also be used for the preparation of pharmaceutical compositions for the treatment of schizophrenia. The pharmaceutical compositions can be in form of tablets, capsules, oral solution, etc.

In the following section, the present invention is further illustrated by examples. They should in no case be interpreted as a limitation of the scope of the invention as defined in the claims.

### Examples

### HPLC characterisation method

Purity or impurity concentrations are expressed in % and refers to area % determined by the HPLC method described herein.

The relative retention time (RRT) values expressed herein are specific to the HPLC conditions described below.

Purity area % is determined by HPLC using a Ace 5 C18 (0.15 m x 4.6 mm x 5 µm packing) analytical column at 40 °C and an eluent flow rate of 0.6 ml/min. Detection is carried out using a UV detector operating at 280 ηm. Elution conditions are **characterized in that** the injection volume is 5 µl and the mobile phase comprises a mobile phase A, consisting of a buffer and mobile Phase B, comprising a methanol.

The gradient program used herein is described below:

| **Time (min.)** | **Mobile Phase A%** |
|---|---|
| 0.00 | 80% |
| 30.00 | 20% |
| 40.00 | 20% |
| 40.01 | 80% |

### Sample preparation.

The sample concentration for assay determination is 1 mg/ml of sample in methanol.

### Buffer Preparation

2.72 g of potassium dihydrogen phosphate are diluted in 1L of purified water. Afterwards, 1 ml of triethylamine (HPLC grade) is added to adjust the pH of the solution to 7.00 ± 0.05.

### Example 1.- Synthesis of crude Paliperidone

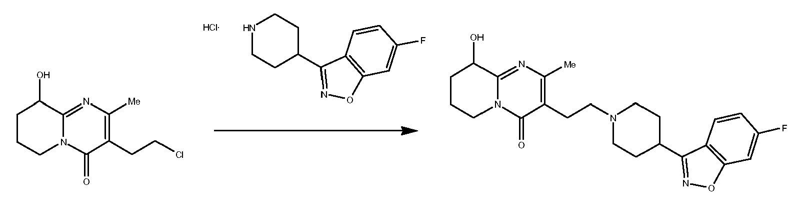

100 g of 3-(2-Chloroethyl)-6,7,8,9-tetrahydro-9-hydroxy-2-methyl-4H-pyrido[1,2-a]pyrimidin-4-one and 106 g 6-fluoro-3-(4-piperidinyl)-1,2-benzisoxazole monohydrochloride are mixed in 2L round bottom flask followed by addition of 500 ml and 176 ml of MeOH and DIPEA respectively. The subsequent suspension is heated under reflux for a period of 24 hours. Once the reaction is complete the temperature is dropped to 0 °C. The precipitate obtained is filtered out and washed with MeOH and further washed with water. 164 g of Paliperidone with a purity of about 98,5 % (by HPLC) are produced after being dried in the oven. Yield 93-94 %.
M.p.=178-181 °C

### Example 2.- Synthesis of Paliperidone hydrochloride

40.0 g of crude Paliperidone and 120 ml of H₂O are incorporated into a 250 ml round bottom flask. The mixture is cooled down to 5-10 °C and a solution of HCl_{aq} (37%) is added. A precipitate is formed and kept at low temperature (5-10 °C) for one 1h. The solid Paliperidone hydrochloride is filtered, washed with cold water and dried. 41,9 g of Paliperidone hydrochloride with a purity between about 99,8% are obtained. Yield of the reaction 96,5%
Mp: 276°C (DSC)

Optionally, the purification of Paliperidone Hydrochloride is carried out by preparing a slurry of Paliperidone salt in 2,5 vol of water at 20°C. The Paliperidone is further filtered and washed with water. The purity of Paliperidone salt obtained when the purification process of Paliperidone Hydrochloride is completed is of about 99.8%.

### Example 3.- Synthesis of pure Paliperidone from Paliperidone hydrochloride

1.0 g of Paliperidone hydrochloride, 20 ml THF and 3 ml H₂O were mixed in a 50 ml round bottom flask. A 20% aqueous solution of NaOH was slowly added until a pH between about 9-10 was reached. The aqueous phase is separated and washed with two portions of 20 ml of THF. The organic phase is collected and treated with activated carbon at 60°C. The obtained reaction mass is filtered, while still hot, and let cool down. Afterwards, the precipitate is further washed with THF. Yield: 75%; Purity 99,9% (by HPLC).

### Example 4.- Synthesis of pure Paliperidone from Paliperidone hydrochloride

20 ml THF, 3 ml H₂O and 1.0 g of Paliperidone hydrochloride were mixed in a 50 ml round bottom flask. A 20% aqueous solution of NaOH was slowly added until a pH between about 9-10 was reached. The aqueous phase is separated and washed two portions of 20 ml of THF. The organic phase is collected and treated with activated carbon at 60°C. The obtained reaction mass is filtered, while still hot. Afterwards, the remaining solvent is distilled off with the purpose of increasing the concentration of the Paliperidone in THF, followed by precipitation of the product, at 0 °C, from isopropyl alcohol. The obtained precipitate is further washed with isopropyl alcohol. The yield was 92% with a purity of 99,9% (by HPLC).

In the following table an example of impurities profile is shown:

| **Compound** | **Retention time/min** | **Crude Paliperidone/%** | **Pure Paliperidone/%** |
|---|---|---|---|
| Unknown | 10.6 | 0.05 | n.d. |
| 9205 | 14,6 | 0.94 | n.d. |
| MP3 | 15,8 | 0.42 | n.d. |
| NO-PLP | 17,7 | n.d. | n.d. |
| NO-PLP | 20,2 | n.d. | n.d. |
| PLP-ceto | 26.2 | 0.04 | 0.04 |
| Paliperidone | 26,7 | 98.19 | 99.91 |
| Unknown | 27,5 | 0.02 | n.d. |
| Unknown | 29.4 | 0.14 | 0.05 |

### Impurities and abbreviations.

9205 and MP3 are reactants. 9205 refers to: 3-(2-Chloroethyl)-6,7,8,9-tetrahydro-9-hydroxy-2-methyl-4H-pyrido[1,2-a]pyrimidin-4-one and MP3 refers to: 6-fluoro-3-(4-piperidinyl)-1,2-benzisoxazole monohydrochloride

NO-PLP refers to: 3-[2-[4-(6-fluorobenzo[d]isoxazol-3-yl)-1-oxypiperidin-1-yl]ethyl]-7-hydroxy-4-methyl-1,5-diazabicyclo[4.4.0]deca-3,5-dien-2-one, as described in US patent 2008171876. NO-PLP shows two retention times since each of them corresponds to each enantiomer
n.d.: not detectable.

## Claims

1. A process for the purification of Paliperidone, which comprises the steps of:
A- Forming a Paliperidone salt from Paliperidone base by reaction of an acid with crude Paliperidone base.
B- Optionally, recovering Paliperidone salt of formula **II**
C- Neutralising Paliperidone salt by treating with a base until a pH between about 9-10 is reached,
D- Extracting Paliperidone base with organic solvent,
E- Collecting Paliperidone,
F- Optionally washing, re-precipitating or re-crystallising the purified Paliperidone.

2. The process of claim 1 **characterised in that** the acid is an inorganic acid selected from hydrochloric acid, hydrobromic acid, sulfuric acid, nitric acid, phosphoric acid and mixtures thereof, preferably hydrochloric acid.

3. The process of claim 1 **characterised in that** the acid is an organic acid selected from acetic acid, citric acid, fumaric acid, formic acid, gluconic acid, lactic acid, oxalic acid, tartaric acid.

4. The process of claim 1 **characterised in that** the base is an inorganic base selected from alkoxides or carbonates of alkaline metals or mixtures thereof.

5. The process according to claim 1, **characterised in that** the base is an organic base selected from ammonia, n-butyl amine, pyrrolidine, diethyl amine, morpholine, piperazine, di-isopropyl amine, triethyl amine, diisopropylethyl amine or mixtures thereof.

6. The process according to claim 1, **characterised in that** the solvent system in step C comprising a first solvent and a second solvent. The first solvent being an organic solvent and the second solvent a non-miscible or partially miscible solvent. The preferred organic solvents are selected from aromatic organic solvents, chloro-compounds, ketones, esters, ethers and mixtures thereof. Preferred second solvent is water.

7. A process according to claim 1, **characterised in that** the organic solvent used in step D is selected from the group of aromatic organic solvents, chloro-compounds, ketones, esters, ethers or mixtures thereof.

8. The process of claim 1, **characterised in that** the re-precipitation and re-crystallisation of step F is carried out in isopropyl alcohol, ethanol, methanol, water, ketones, THF or mixture thereof.

9. Paliperidone according to any of the preceding claims having a total purity of about 99.2 % to about 99.9 %, preferably Paliperidone according to claim 1 having a total purity of about 99.5 % to about 99.9 %

10. Acid addition salt of Paliperidone of formula **II**: 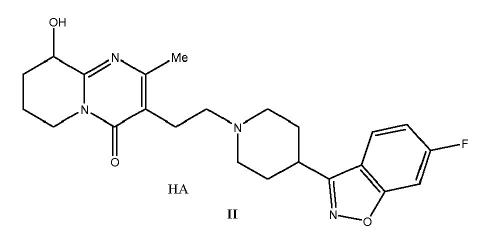 Wherein HA is an organic or inorganic acid selected from the group consisting of: hydrochloric acid, hydrobromic acid, sulfuric acid, nitric acid, phosphoric acid and chromic acid, acetic acid, citric acid, formic acid, gluconic acid, lactic acid, oxalic acid, tartaric acid.

11. Paliperidone Hydrochloride

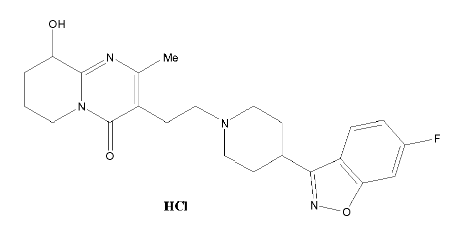

12. Paliperidone free of 3-{2-[4-(6-Fluoro-benzo[d]isoxazol-3-yl)-piperidm-1-yl]-ethyl}-2-methyl-7,8-dihydro-6H-pyrido[1,2-a]pyrimidine-4,9-dione (PLP-ceto).
